# FASCICULE DE BREVET EUROPEEN

(11) **EP 0 781 137 B1**
(45) Date de publication et mention de la délivrance du brevet: **06.09.2006**
(21) Numéro de dépôt: 96924920.0
(22) Date de dépôt: 02.07.1996
(51) Int. Cl.: A61K 31/66, A61K 31/685

(54) **UTILISATION DE PHOSPHOGLYCERIDES COMPRENANT DES PHOSPHOGLYCERO-ETHERS DANS LE TRAITEMENT DE MALADIES NEURO-DEGENERATIVES CHEZ DES ENFANTS ATTEINTS D'HANDICAPS MENTAUX SEVERES**
VERWENDUNG VON PHOSPHOGLYZERIDEN ENTHALTEND PHOSPHOGLYCERO-ETHER ZUR BEHANDLUNG NEURODEGENERATIVER ERKRANKUNGEN IN GEISTIG SCHWER BEHINDERTEN KINDERN
USE OF PHOSPHOGLYCERIDES COMPRISING PHOSPHOGLYCERO ETHERS FOR TREATING NEURODEGENERATIVE DISEASES IN MENTALLY SEVERE HANDICAPPED CHILDREN

(30) Priorité: 07.07.1995 FR 9508223
(43) Date de publication de la demande: 02.07.1997
(73) Titulaire: Institut de Recherche Biologique S.A., 78340 Les Clayes-sous-Bois (FR)
(72) Inventeur: FORGEOT, Marcel, 78720 Dampierre-en-Yvelines (FR)
(74) Mandataire: Burtin, Jean-François
(86) Numéro de dépôt international: PCT/FR1996/001028
(87) Numéro de publication internationale: WO 1997/002828

(56) Documents cités:
- WO-A-93/21190
- DE-A- 4 133 080
- FR-A- 2 721 516
- US-A- 4 613 621

## Description

La présente invention se rapporte au domaine de la chimie thérapeutique et plus particulièrement à celui des phosphoglycérides.

Elle a plus précisément pour objet l'utilisation de phosphoglycérides contenant de 5 à 25% d'éther lipides totaux (plasmalogènes) extraits de cerveaux ou de moelle épinière de mammifères ou de poissons ainsi que d'oeufs de poules, en vue de la réalisation d'un médicament destiné à traiter des maladies neurodégénératives sélectionnées parmi le Syndrome de Down, la maladie de Little, l'état post-ictère nucléaire, la débilité endogène, le grand-mal et le petit-mal chez des enfants atteints d'handicaps mentaux sévères.

Elle a spécifiquement pour objet l'utilisation de pharmaceutiques contenant des phosphoglycéro éthers ou plasmalogènes, associés ou non à des phospholipides, en mélange ou en dilution, dans des excipients inertes, non toxiques, convenant pour l'administration digestive ou parentérale.

On définit les phospholipides comme étant des esters constitués d'une molécule de glycérol estérifié en position 1 et 2 par des chaînes d'acides gras, saturés ou non saturés, et en position 3 par un dérivé d'acide phosphorique. La plupart des phospholipides naturels contiennent des chaînes d'acides gras saturés comme l'acide stéarique ou l'acide palmitique. Par ailleurs, le groupe phosphorique est lié à une chaîne aminoalcoyle pour former la phosphatidylcholine, la phosphatidyl éthanolamine, la phosphatidyl sérine et le phosphatidylinositol. Ces composés qui forment la classe des lécithines ont principalement une valeur alimentaire.

Les phospholipides, selon l'invention, se caractérisent par le fait que le glycérol est estérifié et/ou éthérifié par des chaînes grasses non saturées.

Des essais antérieurs ont montré l'intérêt des phospholipides contenant des acides gras polyinsaturés comme groupement estérifiant.

Leur rôle en nutrition et en diététique a déjà fait l'objet du dépôt de plusieurs demandes de brevet selon qu'ils ont pour origine, les tissus cérébraux ou bien qu'ils soient extraits du jaune d'oeuf de poules dont la nourriture a été modifiée.

Ces phospholipides sont en effet porteurs de chaines d'acide gras à nombre de carbone très élevé et comportent un degré d'insaturation très élevé de la série n-3, en particulier l'acide en C₂₂:6.

La supériorité du rôle nutritionnel des phospholipides cérébraux par rapport à d'autres sources d'acide gras en n-3 a été plus particulièrement démontrée (J.M BOURRE J.Neur.Chem. 60 (1993) 2018-2028).

La présente invention concerne donc l'utilisation sous forme de compositions pharmaceutiques où les phosphoglycérides sont remplacés partiellement ou d'une manière prépondérante par des plasmalogènes dont la formule est 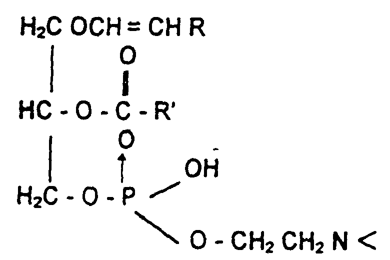 dans laquelle R est un reste alcoyle et de préférence, un radical alcényle ayant une ou plusieurs double liaisons, au plus 5 double liaisons et de 10 à 24 atomes de carbone
R' est une chaîne alcoyle, linéaire ou ramifiée, grasse, non saturée, ayant de 10 à 24 atomes de carbone et de 1 à 6 double liaisons
et l'atome d'azote est engagé dans une structure secondaire, tertiaire ou quaternaire, linéaire ou cyclique.

L'Index Merck (1989) page 1194, définit les plasmalogènes comme des lipides aldéhydogéniques extraits du règne animal et rien n'est connu de leur utilité thérapeutique.

Les phosphoglycéro éthers de l'invention semblent avoir une forte affinité pour les acides gras polyinsaturés à longue chaîne de la série n-3 et on peut en déduire une grande importance physiologique au niveau des eicosanoïdes. Dans ce cas, le plasmalogène présente la particularité de contenir deux fois plus d'un acide gras polyinsaturé (le DHA C₂₂:6 n-3) que les autres phospholipides.

Les molécules de plasmalogènes sont présentes essentiellement au niveau des membranes et semblent douées de remarquables qualités d'échanges membranaires. Elles auraient donc une plus grande activité métabolique au niveau de la reconstruction des membranes.

Il a, par ailleurs, été montré que des cellules mutantes en cultures qui ne synthétisent pas de plasmalogène sont très sensibles aux agents générateurs de radicaux libres (O.MORAND J.Biol.Chem. 263 (1988) 11.797). Ceci laisse supposer un effet protecteur du plasmalogène vitamine E like avec un rôle d'antioxydant dans les stress oxydatifs (B.ENGELMANN Biochem.Biophys. Res. Comm. 204 (1994) 1235).

L'importance des phosphoglycéro-éthers apparaît lors de diverses maladies génétiques, aussi bien chez l'animal que chez l'homme. Ainsi, entre autres, des races de souris mutantes, utilisées en laboratoire, telles que les souris « Jimpy » et les souris « Quaking » ont un taux abaissé de plasmalogènes, aussi bien dans le cerveau que dans la moelle épinière (M.H HACK J. of Chromatog. 145 (1978) 307), de même des porcs atteints de tremblements congénitaux (D.S PATTERSON J.Neurochem. 21 (1973) 397).

Dans cette maladie, on trouve dans le cerveau des enfants atteints, une grande quantité de phospholipides esters avec de très longues chaînes d'acides gras de la série n-6 avec parallèlement une baise très importante du taux d'acides gras de la série n-3 et en particulier, de C₂₂-6 (DHA) (A.POULOS Biochem. J. 253 (1988) 645).

D'autres maladies héréditaires induisent des troubles neurologiques de démyélinisation avec baisse importante du taux de plasmalogène au niveau cérébro-spinal; c'est le cas de la maladie leucodystrophique de Pelizaeus-Merzbacher (J.M BOURRE Europ. Neurol. 17 (1978) 317)., mais aussi la maladie infantile de Refsum, la chondrodysplasie punctata et d'autres (A.K HAJRA Plenum Press (1988) 369-380).

D'autres maladies neurodégénératives sont accompagnées d'une perturbation du métabolisme des phospholipides et, en particulier, du plasmalogène au niveau cérébro-spinal; c'est le cas de la sclérose en plaque (J.M BOGGS Neuro.Chem.Res. 7 Août 1982 -p.953-964).

Dans diverses maladies de démyélinisation et dans l'ischémie, on note une élévation du taux de plasmalogénase dans les tissus cérébraux, ce qui confirme le rôle des plasmalogènes dans la myéline (L.A HORROCK Adv.Exp.Med.Biol. 100 (1978) 423-438).

La dégradation des membranes de phospholipides semble être associée à la maladie d'Alzheimer et on enregistre une baisse du taux de phosphatidylcholine et de phosphatidyl-éthanolamine cérébrales (R.M NIBCH Proc.Natl.Acad.Sci. USA 89 - (1992) 1671), ainsi qu'une baisse du taux d'acides gras polyinsaturés et, en particulier, C₂₂:6, (M.SODERBERG Lipids 26 (1991) 421-425).

Des modifications du même ordre ont été signalées dans le syndrome de Down (B.W BROOKSBANK Mal. Chem. Neuropathol. 11 (1989) 157-185).

Les informations qui précèdent ont incités les Demandeurs à rechercher des sources de phospholipides éthers et à mesurer leur éventuelle efficacité protectrice vis-à-vis des agents oxydants ou des processus dégénératifs.

Le cerveau de mammifères et la moelle épinière apparaissent comme les meilleures sources de phospholipides éthers, sous forme de plasmalogènes; la préparation utilisée selon l'invention contient environ 12 % de plasmalogène avec une forte proportion d'acides gras polyinsaturés n-3, dont la composition est la suivante :

| | |
|---|---|
| sphingomyéline | 5 à 10 % |
| phosphatidylcholine | 20 à 30 % dont 1/20è sous forme de plasmalogènes |
| phosphatidylsérine | 15 à 20 % |
| phosphatidylinositol | 3 à 5 % |
| phosphatidyl éthanolamine | 30 à 40 % dont les 2/3 sous forme de plasmalogènes |
| acide phosphatidique | 3 à 5 % |
| lysophospholipides | 2 à 10 % |
| dont plasmalogènes totaux | 10 à 15 % |
| Somme des acides gras n-3 | = 11 % |
| Rapport n-6/n-3 | = 15 % |
| Rapport C₂₂: 6 (n-3) /total des acides gras | = 10 % |

Par comparaison, les oeufs de poules nourries avec une alimentation riche en acides gras de la série n-3 contiennent une proportion très inférieure de plasmalogènes ainsi qu'une quantité moindre d'acides gras à longue chaîne de la série n-3 comme en témoigne le Tableau I ci-après :

| PHOSPHOLIPIDES D'OEUFS DE POULE | | ETHERS LIPIDES |
|---|---|---|
| Phosphatidylcholine | 70 % | 0 |
| Phosphatidyléthanolamine | 16 % | 2,8 à 4 % |
| Autres phospholipides | 12 % | 0 |
| Total éthers lipides | | 0,5 à 1 % |
| Somme des acides gras n-3 | 6 à 7 % | |
| Rapport n-6/n-3 | 5 à 6 % | |
| Rapport C₂₂:6 (n-3)/ | 5 % | |
| Total acides gras | | |

C'est pourquoi on utilise de préférence une préparation de phospholipides cérébraux riche en plasmalogènes (phosphoglycéro-éthers) dont la composition et la teneur en plasmalogènes est apparue comme plus particulièrement appropriée.

D'une manière plus particulière, les phospholipides utilisés dans la présente invention renferment de 5 à 25 % de plasmalogènes totaux et de préférence de 10 à 15 %.

La présente invention a pour objet l'utilisation de phosphoglycérides contenant de 5 à 25% d'éther lipides totaux (plasmalogènes) extraits de cerveaux ou de moelle épinière de mammifères ou de poissons ainsi que d'oeufs de poules, en vue de la réalisation d'un médicament destiné à traiter des maladies neurodégénératives sélectionnées parmi le Syndrome de Down, la maladie de Little, l'état post-ictère nucléaire, la débilité endogène, le grand-mal et le petit-mal chez des enfants atteints d'handicaps mentaux sévères.

Les compositions utilisées selon l'invention sont destinées à l'administration par voie digestive ou parentérale. Pour ces fins, elles seront additionnées d'excipients ou de diluants appropriés pour ces voies comme des produits minéraux inertes, par exemple le carbonate de calcium, le phosphate tricalcique, le phosphate de magnésium, l'alumine, la silice colloïdale, le kaolin, les argiles, le silicate d'aluminium, le silicate de calcium ou l'oxyde de fer pour la voie digestive, l'eau ou les liquides aqueux pour la voie parentérale.

Les compositions utilisées selon l'invention peuvent également contenir des supports inertes de nature organique comme les amidons, les dextrines, le lactose, la cellulose, les dérivés synthétiques de cellulose, les alginates, les carraghénates, les acides gras, les cires ou les résines.

Les compositions utilisées selon l'invention peuvent également contenir d'autres principes actifs d'action complémentaire comme les vitamines du groupe B (vitamine B1, vitamine B2, vitamine B6, acide folique, acide pantothénique, acide pangamique, vitamine PP), ou bien des sels minéraux (sels de magnésium) ou encore des oligo éléments tels que le selenium, le lithium ou le rubidium.

Les compositions utilisées selon l'invention se présentent donc sous forme d'ampoules buvables, de flacons, de gélules, de capsules, de comprimés nus ou enrobés, de tablettes, de pastilles, de granulés ou de poudres aromatisées ou non, édulcorées ou non.

Les compositions utilisées selon l'invention peuvent aussi se présenter sous forme liquide comme par exemple une préparation gélifiée ou une suspension buvable ou bien encore une émulsion huile dans l'eau.

La présente invention est également décrite avec plus de détails dans les exemples qui suivent :

### EXEMPLE I

Préparation d'une composition à base de phospholipides riche en éther lipides (Plasmalogène) et de préférence extraite de cerveaux ou de moelle épinière de mammifères ou de poissons, et eventueliement d'oeufs de poules

Phospholipides cérébraux 10 à 300 mg dont 10 à 15% d'éther lipides :
- Vitamine B1 0,30 à 4,2 mg
- Vitamine B2 0,30 à 4,8 mg
- Vitamine B6 0,35 à 6 mg
- Acide folique 30 à 600 µg
- Vitamine B12 0,15 à 3 µg
pour une gélule

### EXEMPLE II

Gélules à base de phospholipides cérébraux riches en éther lipides (plasmalogène)
- Préparation selon l'exemple I 70, 0 g
- Lactose 175,5 g
- Vitamine E 20,0 g
- Cellulose microcristalline 30,0 g
- Stéarate de calcium 4,5 g
pour 1000 gélules

### EXEMPLE III

Gélules à base de phospholipides cérébraux riches en éther lipides (plasmalogène)
- Préparation selon l'exemple I 65,0 g
- Oxyde de magnésium 75,0 g
- Gluconate de zinc 100,0 g
- Maltodextrine 59,25 g
- Silice colloïdale 0,75 g
pour 1000 gélules

### EXEMPLE IV

Gélules à base de phospholipides cérébraux riches en éther lipides (plasmalogène)
- Préparation selon l'exemple I 90,5 g
- Oxyde de magnésium 100,0 g
- Autolysat de levure titré en vitamines du groupe B 55,0 g
- Carboxyméthyl cellulose 53,6 g
- Silice colloïdale 0,9 g
pour 1 000 gélules

### EXEMPLE V

Gélules à base de plasmalogènes
- Préparation selon l'exemple I 100,0 g
- Lactose 770,0 g
- Stéarate de magnésium 20,0 g
- Silice colloïdale 3,0 g
pour 1 000 gélules

### EXEMPLE VI

Comprimés à base de phospholipides cérébraux riches en éther lipides (plasmalogène)
- Préparation selon l'exemple I 95,0 g
- Oxyde de magnésium 250,0 g
- Sorbitol 637,5 g
- Cellulose microcristalline 15,0 g
- Silice colloidale 2,5 g
pour 1 000 gélules

### EXEMPLE VII

Ampoules buvables à base de phospholipides cérébraux riches en éther lipides (plasmalogène)
- Préparation selon l'exemple I 45,0 g
- Glycyrrizinate d'ammonium 12,5 g
- Eau purifiée 10 l
pour 1 000 ampoules de 10 ml

### EXEMPLE VIII

Sachet de poudre buvable à base de phospholipides cérébraux riche en éther lipides (plasmalogène)
- Préparation selon l'exemple I 100,0 g
- Oxyde de magnésium 500,0 g
- Gluconate de zinc 125,0 g
- Vitamine E 30,0 g
- Maltodextrine à fort pouvoir d'absorption 2,5 g
- Diluant q.s
pour 1 000 sachets de 5 g

### EXEMPLE IX

Suspension injectable à base de phospholipides cérébraux riches en éther lipides (plasmalogène)
- Phospholipide cérébraux 10 mg
- Chlorure de sodium 15 mg
- Eau PPI QS
pour 1 ampoule de 5 ml

### EXEMPLE X

Le dosage des éthers lipides dans l'extrait de cervelles de mammifères a été réalisé par deux techniques :

### A) Technique chromatographique bidimensionnelle

Dans un premier temps, extraction des lipides totaux par la méthode de Folch, telle qu'elle est décrite par B.Entressangles, dans « Manuel des corps gras » (éditions Lavoisier - Paris) 1992 - p. 1414-1418.
Les éthers lipides sont séparés des phospholipides totaux par chromatographie bidimensionnelle avec hydrolyse intermédiaire aux vapeurs de CIH : les éthers lipides ainsi hydrolysés en lysodérivés correspondants se séparent des phospholipides diacylés au cours de la deuxième dimension.

### B) Par spectrométrie NMR

Les phospholipides sont quantifiés par spectroscopie [³¹P] RMN en employant le triphényl phosphate comme témoin interne. Les échantillons sont traités d'une manière spéciale pour obtenir des lignes et une bonne résolution. Les valeurs résultantes sont des % en moles de phospholipide par rapport au standard interne.
Pour transformer le % en mole en % en poids, les valeurs sont multipliées par le poids moléculaire du phospholipide correspondant.

Le cholestérol et les cérébrosides sont déterminés d'une manière quantitative par spectroscopie [¹H]RMN en ajoutant les standards internes correspondants. Les données sur les éthers lipides sont obtenues à partir de signaux inclus dans les trois spectres. Les acides gras libres sont calculés à partir du spectre RMN du [¹³C]. En supplément aux mesures par [³¹P] RMN, on a effectué des spectres [¹H] RMN et un spectre [¹³C] RMN. On a ajouté du cholestérol et des cérébrosides comme standard pour obtenir des valeurs quantitatives sur ces deux types de lipides par l'intermédiaire du spectre [¹H] RMN. En utilisant le spectre [¹³C] RMN, on peut calculer la teneur en acides gras libres et en alcenyléthers. Le tableau ci-dessous montre les valeurs obtenues sur les phospholipides cérébraux.

| | | |
|---|---|---|
| Somme phospholipides | 53,6 % | |
| - PC-Ether | | 0,7 % |
| - PE-Ether | | 12,0 % |
| Cholestérol | 7,0 % | |
| Cérébrosides | 9,0 % | |
| Acides gras libres | 5,0 % | |
| Somme | 74,6 % | |

PC Ether = phosphatidyl choline éther
PE Ether = phosphatidyl éthanolamine éther

### EXEMPLE XI

### Préparations des éthers de lipides à partir de tissus cérébraux de porc

Les phospholipides cérébraux selon l'invention sont obtenus par épuisement de cervelles de porc prélevées sur des animaux fraîchement abattus selon les modalités suivantes :
- les cervelles de porc sont prélevées sur des animaux fraîchement abattus en provenance d'élevages exempts de toute maladie infectieuse et suivis rigoureusement sur le plan sanitaire par les services vétérinaires.
- les cervelles sont immédiatement congelées à -20°C et conservées à cette température
- les cervelles sont ensuite ramenées à une température comprise entre -5° et 0°, avant passage dans un hachoir industriel et des broyeurs, de façon à obtenir une pâte liquide dont la teneur en eau est d'environ 80%.
- La pâte de cervelle est transférée au sommet d'une chambre d'atomisation dans laquelle l'eau est immédiatement évaporée dans un courant d'air chaud à 190/195°C.
- la poudre obtenue est introduite dans un réacteur contenant un mélange d'hydrocarbures aliphatiques à base d'hexane et maintenue sous agitation.
- après filtration, la phase liquide est concentrée sous vide et donne naissance à un extrait brut
- l'extrait brut est ensuite coulé dans l'acétone en présence d'un antioxydant alimentaire
- le précipité obtenu est filtré sous pression d'azote
- le produit recueilli est séché sous vide et contient les phospholipides cérébraux purifiés

### EXEMPLE XII

Démonstration de l'effet pharmacologique des préparations selon l'invention :

On a utilisé une préparation de phospholipides cérébraux, riche en plasmalogènes (phosphoglycéro-éther) comme agent protecteur dans un certain nombre de tests chez l'animal.

### a) sur les névrites expérimentales provoquées par la pyrithiamine

La pyrithiamine est un antimétabolite qui, à la dose de 5 mg/kg I.P, provoque chez la souris une hyperpolarisation tétanique.

L'activité protectrice des phospholipides a été testée d'une part en traitement préventif, c'est-à-dire administrés en même temps que la pyrithiamine et d'autre part en traitement curatif, c'est-à-dire lorsqu'apparaissent les premiers signes neurologiques.

Le tableau ci-après fait état des résultats.

On constate que l'activité protectrice en traitement préventif est totale jusqu'à des doses aussi faibles que 2,5 mg de phospholipides pour 10 g d'aliments.
Si on sait qu'une souris de 20 g consomme environ 3 g d'aliments par jour, la dose efficace est de l'ordre de 0,35 mg de phospholipides riches en plasmalogènes selon l'invention, par animal.

| Doses de phospholipides dans 10 g d'aliments dans les groupes expérimentaux | Effet préventif | | Effet curatif | |
|---|---|---|---|---|
| | Témoins | Experim. | Témoins | Experim. |
| 10 mg | +++ | 0 | +++ | + |
| 5 mg | +++ | 0 | +++ | ++ |
| 2,5 mg | +++ | 0 | +++ | ++ |
| 2 mg | +++ | + | +++ | ++ |
| 1 mg | +++ | +++ | +++ | +++ |

| | | | | |
|---|---|---|---|---|
| +++ Polynévrites aiguës : tous les animaux meurent en quelques jours | | | | |
| ++ Symptômes atténués : survie pour plus de 50 % des animaux | | | | |
| + Symptômes légers : tous les animaux survivent | | | | |
| 0 aucun symptôme | | | | |

### b) effet protecteur des phospholipides riches en plasmalogène sur les manifestations neurologiques provoquées par l'oxotrémorine

L'oxotrémorine provoque chez la souris des tremblements statiques très caractéristiques, de type parkinsonien, ainsi que des symptômes périphériques tels que lacrimation et salivation.

| Doses de phospho-lipides dans 10 g d'aliments pour les groupes expérimentaux | Durée du traitement avec phospholipides avant oxotrémorine | Intensité des symptômes en fonction du temps après arrêt du traitement | | | |
|---|---|---|---|---|---|
| | | 1j | 2j | 3j | 4j |
| 0 mg | | +++ | +++ | | |
| 50 mg | 3 jours | 0 | 0 | ++ | |
| 30 mg | 3 jours | 0 | 0 | ++ | |
| 20 mg | 3 jours | 0 | ++ | +++ | |
| 10 mg | 3 jours | 0 | +++ | | |
| 0 mg | | +++ | +++ | | |
| 50 mg | 1 jour | 0 | 0 | +++ | |
| 30 mg | 1 jour | 0 | 0 | +++ | |
| 20 mg | 1 jour | 0 | +++ | | |
| 10 mg | 1 jour | 0 | +++ | | |

Le tableau montre clairement que 50 mg de phospholipides pour 10 g d'aliments, administrés pendant 3 jours avant l'injection d'oxotrémorine inhibent complètement l'apparition des symptômes (tremblements); cette inhibition à l'action d'oxotrémorine dure 3 jours.

### c) effets des phospholipides riches en plasmalogènes sur l'encéphalomyélite allergique expérimentale

Chez 20 rats, on induit une encéphalomyélite par injection d'une suspension de moelle épinière (adjuvant de Freund). La maladie se développe du 10ème au 14ème jour.
Ces animaux répartis en trois groupes, ont été ensuite traités chaque jour par des doses croissantes de phospholipides.

| Doses de phospholipides cérébraux I.M | Absence de symptômes | | | Non guéris |
|---|---|---|---|---|
| | après 7 j | après 14 j | après 21 j | |
| 0,01 mg | 1 | 2 | 1 | 16 |
| 0,02 mg | 8 | 17 | | 3 |
| 0,5 mg | 6 | 18 | | 2 |

L'effet curatif est donc très net.

### EXEMPLE XIII

### Démonstration de l'effet clinique des préparations selon l'invention

Un essai dinique a été entrepris auprès d'un groupe d'enfants atteints d'handicaps mentaux sévères. Un groupe traité de 104 enfants a été comparé à un groupe non traité de 85 enfants de 1 à 14 ans.
Le traitement a consisté en un apport de 30 mg de phospholipides par jour per os pendant des périodes de 6 à 12 mois selon les cas. Des tests psychométriques, la mesure du Q.I ou l'enregistrement électroencéphalographique ont été réalisés suivant l'âge ou la symptomatologie.

On remarquera dans le tableau ci-après, que plus de six enfants sur dix on retiré un bénéfice important de ce traitement.

| | RESULTATS ENFANTS TRAITES | | | | | RESULTATS ENFANTS NON TRAITES | | | | |
|---|---|---|---|---|---|---|---|---|---|---|
| | Nbre | Nul | Faible | Important | Considérable | Nbre | Nul | Faible | Important | Considérable |
| Syndrome de DOWN | 40 | O | 13 | 21 | 6 | 24 | 6 | 15 | 3 | 0 |
| Maladie de LITTLE | 22 | 6 | 6 | 8 | 2 | 11 | 9 | 2 | 0 | 0 |
| Etat post méningo-encéphalltique | 7 | 0 | 3 | 4 | 0 | 7 | 5 | 2 | 0 | 0 |
| Etat post lctère-nucléaire | 9 | 0 | 3 | 4 | 2 | 5 | 4 | 1 | 0 | 0 |
| Débilité endogène | 13 | 2 | 2 | 6 | 3 | 18 | 11 | 7 | 0 | 0 |
| Petit mal | 4 | 0 | 0 | 3 | 1 | 6 | 3 | 2 | 1 | 0 |
| Grand mal | 9 | 1 | 2 | 4 | 2 | 10 | 6 | 3 | 1 | 0 |
| **TOTAL** | **104** | **9** | **29** | **50** | **16** | **81** | **44 32** | | **5** | **0** |
| | | **8,6 %** | **27,8 %** | **48 %** | **15,3 %** | | **42,3 %** | **30,7 %** | **4,8 %** | |
| | | | | **63,3 %** | | | | | | |

## Revendications

1. Utilisation de phosphoglycérides contenant de 5 à 25% d'éther lipides totaux (plasmalogènes) extraits de cerveaux ou de moelle épinière de mammifères ou de poissons ainsi que d'oeufs de poule en vue de la réalisation d'un médicament destiné à traiter des maladies neuro-dégénératives selectionnées parmi le syndrome de Down, la maladie de Little, l'état post-ictère nucléaire, la débilité endogène, le grand-mal et le petit-mal chez des enfants atteints d'handicaps mentaux sévères, administré par voie digestive ou parentérale.

2. Utilisation selon la revendication 1;
dans laquelle les phosphoglycérides riches en éther lipides (plasmalogènes) contenus dans les phospholipides répondent à la formule : 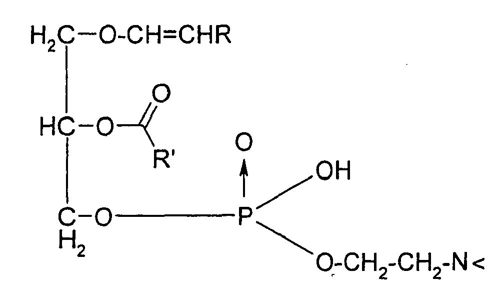 dans laquelle :
- R est un reste alcoyle, et de préférence un radial alkényle ayant une ou plusieurs doubles liaisons, avec un maximum de 5 doubles liaisons et de 10 à 24 atomes de carbone,
- R' est une chaîne alcoyle linéaire grasse, non saturée, ayant de 10 à 24 atomes de carbone et de 1 à 6 doubles liaisons
- et l'atome d'azote est engagé dans une structure secondaire, tertiaire ou quaternaire, linéaire ou cyclique.

3. Utilisation des phosphoglycérides selon la revendication 1 ou 2 répondant à la composition suivante :
| | |
|---|---|
| Sphingomyéline | 5 à 10 % |
| Phosphatidylcholine (dont 1/20 est sous forme de plasmalogènes) | 20 à 30 % |
| Phosphatidylsérine | 15 à 20 % |
| Phosphatidyl inositol | 3 à 5 % |
| Phosphatidyléthanolamine (dont les 2/3 sont sous forme de plasmalogènes) | 30 à 40 % |
| Acide phosphatidique | 3 à 5 % |
| Lysophospholipides | 2 à 10 % |
| avec une teneur en plasmalogènes totaux de | 10 à 15 % |
| Somme des acides gras de la série n-3 | 11 % |
| Rapport n-6/n-3 | 15 % |
| Rapport C₂₂ : 6 (n-3)/total des acides gras | 10 % |

## Claims

1. Use of phosphoglycerids containing from 5 to 25 % of total ether lipids (plasma logens) extracted for brain or marrow of mammals or fished as well as from hour eggs in view of the production of a drug intented for the treatment of neuro degenerative diseases selected from the group consisting of Down's syndrome, little's disease, nuclear frost ictenic conditions, endogenous debiliby, grand mal, petit mal, in children suffering from severe mental handicap.

2. Use according to claim 1 wherein the phosphoglycerids rich in ether lipids (plasmalogens) contained into the phospholipids have the formula: 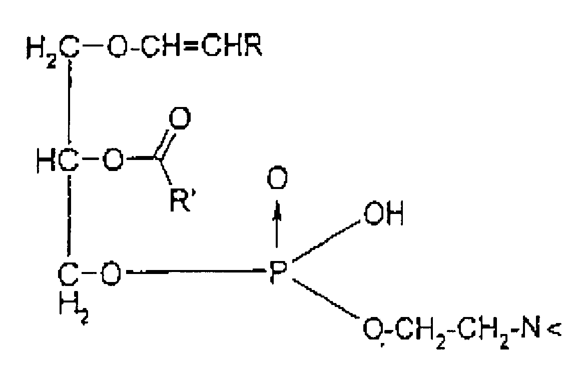 in which
- R is an alkyl chain preferably an alkenyl chain with one or more double bonds with at the maximum 5 double bonds and 10 to 24 carbon atoms.
- R' is a straight, fatty unsaturated chain with 10 to 24 carbon atoms and from 1 to 6 double bonds.
And the quaternary nitrogen atom is inserted in a secondary, tertiary or quaternary straight or cyclic structure.

3. Use of phosphoglycerids according to claim 1 or claim 2 having the following composition:
■ Sphingomyéline 5 to 10 %
■ Phosphatidylcholine (the 1/20 of which is in the form of plasmalogens) 20 to 30 %
■ Phosphatidylsérine 15 to 20 %
■ Phosphatidyl inositol 3 to 5 %
■ Phosphatidyléthanolamin (the 2/3 of which is in the form of plamalogens 30 to 40 %
■ Phosphatidic acid 3 to 5 %
■ Lysophospholipids with a total content in plasmalogens ranging from 10 to 15 %
■ Summ of fatty acids of the serie n-3 11 %
■ Ratio n-6/n-3 15 %
■ Ratio C₂₂ : 6 (n-3)/total amount of fatty acids 10 %

## Patentansprüche

1. Verwendung von Phosphoglyzeriden enthaltend eine Gesamtmenge von 5 bis 25 % Ether-Lipide (Plasmalogenen), extrahiert aus den Gehirnen oder dem Rückenmark von Säugetieren oder Fischen sowie aus Hühnereiern, zur Herstellung eines Medikaments zur Behandlung ausgewählter neurodegenerativer Erkrankungen, darunter das Down-Syndrom, die Little-Krankheit, den Zustand nach Kernikterus, die endogene Debilität, das Grand Mal und das Petit Mal geistig schwer behinderter Kinder, das auf enteralem oder parenteralem Weg verabreicht wird.

2. Verwendung nach Anspruch 1,
wobei die in den Phospholipiden enthaltenen Phosphoglyzeride, die reich an Ether-Lipiden (Plasmalogenen) sind, folgender Formel entsprechen: 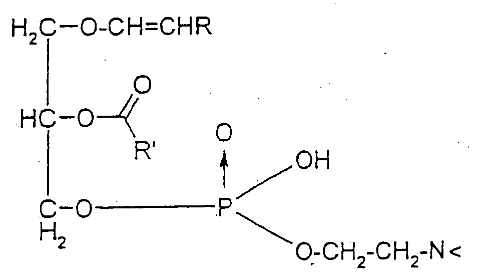 wobei:
- R ein Alkylrest ist, vorzugsweise ein Alkenylradikal mit einer oder mehreren Doppelbindungen mit maximal 5 Doppelbindungen und 10 bis 24 Kohlenstoffatomen,
- R' eine lineare, fette, nicht gesättigte Alkylkette mit 10 bis 24 Kohlenstoffatomen und 1 bis 6 Doppelbindungen ist
- und das Stickstoffatom in eine sekundäre, tertiäre oder quaternäre, lineare oder ringförmige Struktur eingefügt ist.

3. Verwendung der Phosphoglyzeride nach Anspruch 2, die folgender Zusammensetzung entsprechen:
| | |
|---|---|
| Sphingomyelin | 5 bis 10 % |
| Phosphatidylcholin (davon 1/20 in Form von Plasmalogenen) | 20 bis 30 % |
| Phosphatidylserin | 15 bis 20 % |
| Phosphatidylinositol | 3 bis 5 % |
| Phosphatidylethanolamin (davon 2/3 in Form von Plasmalogenen) | 30 bis 40 % |
| Phosphatidsäure | 3 bis 5 % |
| Lysophospholipide | 2 bis 10 % |
| mit einem Gesamtplasmalogengehalt von | 10 bis 15 % |
| Summe der Fettsäuren der Reihe n-3 | 11 % |
| Verhältnis n-6/n-3 | 15 % |
| Verhältnis C₂₂:6(n-3)/Summe der Fettsäuren | 10 % |
